# EUROPEAN PATENT APPLICATION

(11) **EP 2 186 515 A1**
(43) Date of publication of application: **19.05.2010**
(21) Application number: 08778973.1
(22) Date of filing: 04.07.2008
(51) Int. Cl.: A61K 31/55, A61K 31/135, A61P 25/24

(54) **PHARMACEUTICAL COMPOSITION COMPRISING THE COMBINATION OF AN ANTI-DEPRESSIVE AGENT AND AN ANSIOLYTIC AGENT FOR CONTROL AND TREATMENT OF DEPRESSIVE DISORDERS**

(30) Priority: 06.07.2007 MX MX07008324
(71) Applicant: World Trade Import-Export, WTIE A.G., 6302 Zug (CH)
(72) Inventor: GARCIA ARMENTA, Maria Elena, C. P. 45020 Zapopan, Jalisco (MX); SANTOS MURILLO, Josefina, México (MX); ALVAREZ OCHOA, Victor Guillermo, México (MX)
(74) Representative: Carvajal y Urquijo, Isabel
(86) International application number: PCT/MX2008/000083
(87) International publication number: WO 2009/008694

(57) **Abstract**

The present invention concerns a pharmaceutical composition formed by the synergic combination of an anti-depressive selective 5-HT reuptake inhibitor, such as is the active principle: Sertraline and an anxiolytic agent belonging to the triazolobenzodiazepine group, such as is the active principle: Alprazolam, which are formulated in a single dosing unit to be administered by oral means in the form of capsules or tablets, the former being prescribed for the control and treatment of diseases such as: depressive disorders and related pathologies.

## Description

### FIELD OF INVENTION

The present invention is applicable in pharmaceutical industry and describes a pharmaceutical composition comprising a synergistic combination of an 5-HT reuptake antidepressant agent selective inhibitor, such as active principle: Sertraline and an anxiolytic agent belonging to the triazolobenzodiazepine group, such as active principle: Alprazolam; which are formulated in a single dosage unit, which is indicated for control and treatment of depressive disorders and related pathologies.

The combination of above mentioned active principles produces a higher synergistic effect when administered together in a single dosage unit than when independently administered thus generating benefits such as: lower concentrations of formulated active principles, lower administered doses, faster action, higher therapeutic effect efficacy, lower interaction risk among drugs at liver level and lower risks that adverse events are present.

### BACKGROUND OF INVENTION

World Health Organization defines depression as the most common mental disorder. It affects about 340 million people around the globe with a prevalence from 15 to 25%, being highest in women.

Depression etiology is multifactorial and causes may be divided in: biologic, genetic and psychosocial.

Biologic theory reports alterations in biogenic amines, noradrenaline disorders (decrease), serotonin (decrease), dopamine (decrease) and GABA (decrease); in addition to neuroendocrine alterations as adrenal axis and thyroid axis disorders. Genetic theory reports that from 1 to 1.5 times from patients who suffer mood state disorders are more prone to suffer the same disorder in first grade relatives. Psycho-social theory is associated with life events and stressing factors.

Depression is a treatable disease. Many wrongly believe that depression is normal in elder people, youngsters, menopausal women, first-time mothers and in persons who suffer chronic diseases, but this is a wrong concept since depression is never something normal regardless of age, sex or status. Any person who experiences depressive symptoms shall be diagnosed and treated to invert this condition.

Depression is an affective disease which varies from transient mood state lows which are part of life itself until clinical syndrome of significant severity and length with associated signs and symptoms markedly different from normality.

Similarly to other diseases, there are several types of depressive disorders. The three more common depression types are: Major Depressive Disorder, Dysthymic Disorder and Bipolar Disorder. In each of these three types of depression, the number, severity and persistence of symptoms vary.

*Major Depressive Disorder* is characterized by a combination of symptoms, which are below described, which interfere with the capability to perform individual daily activities (working, studying, sleeping, eating) and enjoying activities which formerly were pleasant.

*Dysthymic Disorder* is a less severe depression type, including chronic symptoms (long term) which do not impair a lot; however, they interfere with individual performance and well-being. The essential feature of this disorder is a chronically depressive mood state which is present most part of day in most days at least during 2 years. Many persons with dysthymia may also suffer from severe depressive events at any time in their life.

*Bipolar Disorder* is another type of depression which is not as frequent as other depressive disorders. Bipolar disorder is characterized by cyclic changes in mood state: high or euphoric mood phases (mania) and low mood phases (depression). Changes in mood state may be dramatic and fast, but they are often gradual. When any person is in cycle depressive phase, it may suffer one from several or from all depressive disorder symptoms. When being in maniac phase, the person may be hyperactive, excessively talking and having a great amount of energy. Mania often affects thinking, right mind and the way of behave related with others. This may lead to person to go into severe problems and embarrassing situations, in maniac phase the individual may feel happy or euphoric, with magnificent projects, making wild business decisions or involving in romantic adventures or fantasies.

Not all individuals experience depression in the same way as symptoms vary according to persons. Depression may be classified as mild, moderate or severe depending on symptoms number and severity.

Symptoms of Major Depressive Disorder are: sad, anxious or "empty" persistent mood state, hopelessness and pessimism feelings, guilt feelings, uselessness and helplessness, lack of interest or pleasure in hobbies and activities which formerly were enjoyed, including sexual activity, energy decrease, tiredness, fatigue, feeling of being in "low motion", difficulty in concentrating, remembering and making decisions, insomnia, earlier wake up or longer sleeping, weight, appetite loss or both or on the contrary, eating more and weight increase, death or suicide thoughts or suicide attempts, restlessness, irritability, persistent physical symptoms which do not respond to medical treatment such as headaches, digestive disorders and other chronic aches.

Traits associated with Dysthymic Disorder are similar to those of a larger depressive event. Most frequently found symptoms in dysthymic disorder are: incompetence feelings, generalized loss of interest or pleasure, social isolation, guilt or sadness feelings as to past events, subjective feelings of irritability or excessive anger; activity, efficiency and productivity decrease. In children, dysthimic disorder seems to be similarly present in both sexes and frequently causes impairment in school performance and social interaction. Generally, children and youngsters with dysthymic disorder are irritable and unstable, further being sad. They have a low self-esteem, little social skills and they are pessimist. In adults, women are twice to three times more prone than men to show a dysthymic disorder.

Bipolar Disorder produces pathologic mood changes, from mania to depression, with a tendency to relapse and spontaneously disappear. Both maniac and depressive events may dominate and produce some changes in mood state, or mood state change patterns may be cyclic, often starting with a mania which terminates in a deep depression. Some individuals are called "fast cyclers" since their mood may change several times a day. Others show what is called "mixed states" wherein depressive thoughts may appear in a maniac episode or vice versa. When bipolar disorder is present in children, it generally appears in mixed form.

During depressive phase patient shows: self-esteem loss, lost in thought, hopelessness or impairment feelings, excessive or unsuitable guilt feelings, fatigue (tiredness or boredom) lasting weeks or months, exaggerated slowness (inertness), persistent morning sleepiness, insomnia, concentration problems, easy distraction by non-trascendent events, difficulty in decision making, appetite loss, involuntary weight loss, abnormal thoughts about death, suicide thoughts, suicide planning or suicide attempts, decrease in daily activity interest, decrease in pleasure caused by daily activities.

Maniac phase shows: mood state exaltation, increase in activities oriented towards goals, brief ideas or accelerated thought, high self-steem, less sleep need, agitation, logorrhea (talking more than usual or having a need to continue talking), increase in involuntary activity (that is, walking from one side to the other, hand twisting), excessive restlessness, involuntary weight increase, low temperament control, extreme irresponsibility behavior pattern, increase in activity directed to social or sexual field, excessive and damaging commitment with pleasure activities having a great potential of causing painful consequences (parties, having multiple sexual partners, alcohol and other drug consumption), false beliefs (delirium), hallucinations. Maniac and depressive symptoms may be simultaneously or in a fast succession in the called mixed phase.

Depressed mood, pathologic sadness and interest or satisfaction loss in almost any activity are depression key symptoms. Patients may comment that they are sad, hopeless, or within melancholy.

Major depressive disorder is characterized by one or more major depressive episodes, which are maintained at least during 2 weeks, wherein the depressive mood state or lack of interest or satisfactions in almost any activity is accompanied by at least other four depression symptoms. The essential feature of a major depressive disorder is a manifestation of a clinical profile characterized by one or more major depressive episodes without maniac, mixed or hypomaniac episodes. In order to perform a diagnosis of a major depressive disorder the mood state disorder episodes induced by substances (due to drug, medicament or toxic exposure direct physiological effects) are neither taken into account, nor the mood state disorders due to a medical disease. Moreover, episodes are not better explained by a presence of a schizoaffective episode and they are not overlapped to schizophrenia, a schizophreniform disorder, a delirious disorder or an unspecified psychotic disorder.

Dysthymical disorder is characterized by at least 2 years having more days with depressive mood state than without it, further being accompanied by other depressive symptoms which do not fulfill with the criteria for a major depressive episode.

In some individuals with mild depressive episodes the activity may seem normal, but at the expense of a very significant effort. Patients frequently describe symptoms with an agony-type emotional pain, being occasionally common that individual show inability to cry.

Two thirds of patients suffering from depression think about suicide and 15% of them finally commit it. Almost all patients manifest an energy loss which makes difficult to complete daily tasks, worsening labor and school performance and further decreasing motivation for starting new projects.

80% of patients have difficulty in sleeping, particularly an earlier wake up (terminal insomnia) and multiple sleep interruptions. Some patients show hypersomnia, which in addition to an increase in appetite and weight gain constitute atypical symptoms.

Anxiety is a common symptom (including panic crisis) and usually complicates depressive disorder treatment, in addition to alcohol abuse which is usually present in some patients. 50% of patients get worse in the morning with higher severity in symptoms which are released as day goes by. Many individuals refer or show a high irritability manifested as: persistent anger, tendency to respond to events with fit of anger, insulting others or an exaggerated frustration feeling by things without significance.

More than a sad or discouraged mood state, in children an irritable or unstable mood state may be present. This presentation form shall be distinguished from the "spoiled child" pattern with irritability before frustration.

Individuals with a depressive episode are often present with cry, irritability, sadness, obsessive rumination, anxiety, phobias, excessive concern about physical health and pain complaints.

During a depressive episode some individuals show anxiety crisis with a pattern which fulfills with anxiety disorder criteria. Some individuals refer problems with personal relationships, less satisfactory social interactions or problems with sexual activity.

The most severe consequence of a depressive episode is the suicide attempt or consumed suicide. Suicide risk is especially high for individuals with psychotic symptoms, prior suicide attempt history, consumed suicide or substance concomitant consumption familial history.

Treatment schemes include the use of conventional antidepressants or those corresponding to the group of 5-HT reuptake selective inhibitors; concomitantly associated or administered with triazolobenzodiazepine.

Studies have confirmed that depressive disorders and anxiety disorders coexist in more than 60% of the cases, therefore many patients receive antidepressant-anxiolytic concomitant pharmacotherapy.

### SUMMARY OF INVENTION

In order to provide a pharmaceutical alternative to achieve a better life quality in patients who suffer from depressive disorders and related pathologies, a development for a pharmaceutical composition below described was carried out.

### DETAILED DESCRIPTION OF INVENTION

Most of medicaments currently found in marketplace for depressive disorder treatment comprise active principles which are independently formulated, those which fulfill with a specific therapeutic activity; however, when these medicaments are concomitantly administered and under doses which are usually used they produce negative interaction effects because of their chemical features, metabolism and consequently their high doses, dose frequency and long-term use.

In the light of the above and in order to suppress all the drawbacks which are present when administering independently active principles, the development of a pharmaceutical composition subject of present invention was carried out, which comprises a synergistic combination of a 5-HT reuptake selective inhibitor antidepressant agent and an anxiolytic agent belonging to the group of triazolobenzodiazepines; which produce a satisfactory therapeutic effect when administered orally together in a single dosage unit unlike when these are independently administered, causing benefits such as: lower concentrations of formulated active principles, lower administered doses, faster action, higher therapeutic effect efficacy, lower interaction risk among drugs at liver level and lower risks that adverse effects are present.

**Serotonin reuptake selective inhibitors (ISRS)** are synthetic origin drugs which act as selective blockers of serotonin neuronal reuptake and their chemical structure markedly differs from one substance to others. Serotonin neuronal reuptake inhibiting drugs are very liposoluble molecules, they are efficiently orally absorbed, broadly spreading in organism, strongly bonding with plasma proteins and may displace other drugs such as digoxine, salicilates, dicoumarin anticoagulants and phenothiacines, they are biotransformed in the liver, leading in some cases to an active metabolite with a very prolonged plasma half-life, removal is by renal route but as these substances are metabolized in organism, liver dysfunction cases shall require a dose adjustment. Metabolites derived from fluoxetine, paroxetine, Sertraline and fluvoxamine inhibit some isoenzymes from cytochrome P450 therefore they may block the biotransformation of many drugs.

Selective inhibiting drugs from serotonin neuronal reuptake perform as antidepressant drugs with a clinical efficacy similar to that from tricylic antidepressants since they do not modify the mood state in healthy individuals and they lack of euphoric effects. They have a latency term from 2 to 3 weeks which suggests the existence of receptor adaptive mechanisms in the same way occurring with tricyclic antidepressants. Sertraline, fluoxetine and fluvoxamine produce adrenoceptor β subsensitivity, but this effect is not present with paroxetine which is the most powerful drug from the group.

Serotonin neuronal reuptake selective inhibitors do not induce desensitization with serotoninergic self-receptors, but they facilitate serotoninergic neurotransmission through 5-HT1A post-synaptic receptor sensitization. ISRC antidepressants have analgesic action, which is related with easing antinociception endogenous mechanisms of triptaminergic nature. Serotonin neuronal reuptake selective inhibitors in therapeutic doses practically lack of anticholinergic, antihistaminic and α and β adrenolytic effects.

Selective inhibiting drugs of serotonin neuronal reuptake are used in treatment of major and minor depression, anxiety, panic crisis, agoraphobia, obsessive-compulsive disorders, nervous anorexia, bulimia and Giles de la Tourette syndrome; their use have been also proposed for alcoholism, ludopathies and pharmacodependency. Serotonin neuronal reuptake selective inhibitors are very efficient in all those disorders derived from the lack of control in impulses where serotonin is involved.

**Sertraline** is an antidepressant belonging to the group of 5-HT reuptake selective inhibitors. This agent almost fully lacks of activity over other aminergic neurotransmitter reuptake. It does not have either affinity for post-synaptic adrenoceptors or muscarinic, histaminergic, GABAergic or benzodiazepine receptors.

Sertraline, derived from 1-amino tetrahydronaphtalene, is a solid, crystalline, white-color, hydrosoluble, light-unstable substance. Its empirical formula is C17H17C12N. HCl with a molecular weight of 342.7. It is orally efficiently absorbed, although more slowly than other group agents. A significant part of its pharmacologic activity in the demethylated metabolite is lost. Plasma half-life of sertraline is about 22 to 30 hours, which allows its delivery in a daily single dose without causing a significant accumulation after chronic administration. Plasma half-life is similar after administering a single dose compared with equilibrium state, indicating a linear kinetics in the margin of used doses (50-200 mg daily doses). Sertraline is attached to plasma proteins in 99%.

Similarly as other ISRS, Sertraline is subject to a broad metabolic degradation in liver. Demethyl sertraline is firstly formed by demethylation and then both compounds, Sertraline and demethylSertraline, are metabolized to ketones, hydroxylated derivatives and conjugated with glucuronic acid, and final metabolites are excreted in similar rate in urine an bile. After Sertraline oral administration, 88% of dose is recovered in urine and feces after seven days.

DemethylSertraline shows an activity over serotonin reuptake process eight times lower than unaltered drug, having been tested "in vivo" that Sertraline antidepressant activity is only due to the activity of the drug itself.

Lineal clearance profile of Sertraline determines a lower interindividual variability in achieved concentrations, which facilitates posologic scheme optimization. Thus after delivering fixed doses of Sertraline and other ISRS, the variation coefficient of plasma concentrations was lower for Sertraline (38%) than for others ISRS, due to a lower variability in the clearance process. In studies performed after multiple dose administration, there were no changes found in Sertraline disposal kinetics in elderly. In geriatric patients the dose is not considered to be modified, showing together a suitable kinetic profile for this group of population.

Due to the absence of activity in biotransformation products, it looks foreseeable that no modifications are produced in antidepressant response with a chronic delivery in case of accumulation of metabolites.

Sertraline produces a weak in vitro inhibition of CYP2D6 isoenzyme and possibly also affects to 2C and 3A4. Its activity and that from N-demethylSertraline metabolite, over CYP2D6 isoenzyme is five to ten times lower than that established for fluoxetine and its active metabolite.

**Benzodiazepines (BZD)** are minor tranquilizers. Activity from central nervous system is slowed through their action, that is, messages which enter into and leave from brain are slowed or delayed to the rest of the organism, including physical, mental and emotional responses. Similarly to alcohol and opiates, benzodiazepines are central nervous system depressant drugs.

BZD are produced through chemical synthesis processes. Benzodiazepine group comprises more than 24 different drugs. They are the most commonly prescribed minor tranquilizers by physicians to calm anxiety (anxiolytics) or to promote sedation and sleep (hypnothics).

Benzodiazepines depress mental activity, reduce alert state, relief stress and anxiety in addition to inducing calm and relaxation sensations. These calm sensations may be experienced as pleasant. However in persons who are not anxious, benzodiazepines generally do not produce positive pleasure or wellbeing sensations. This is what mostly explains that they are not popular as recreational drugs. In high doses they function as sleep inducers.

BZD exert their physiological effects through their attachment to a GABA receptor subunit. GABA receptor comprises an ionic channel and several subunits which are fixed to different substances: one subunit is fixed in alcohol, another to benzodiazepines and another to barbiturics. The subunit which is fixed to benzodiazepines has been named: *benzodiazepinic* receptor. Benzodiazepines which increase GABA effect are called agonists. GABA is the major inhibitor neurotransmitter from CNS and GABA synapse, distributed through brain and spinal cord, comprising about 40% of all synapses.

Physiological function of GABAergic synapses is to modulate neuron polarization. GABA receptor achieves it through chlorine channel opening or closure. Chlorine channel opening allows that more chlorine ions enter into the cell. Negatively charged chlorine ion flow increases the electric gradient through cellular membrane and converts the neuron into a less excitable one. Channel closure decreases electric polarization and cell is converted into a more excitable one.

Attachment of an agonist to benzodiazepine receptor facilitates GABA effect (opening chlorine channel). Clinical effects are anxiety reduction, sedation and increase in anticonvulsant threshold.

In order to perform BZD delivery, it is important to know whether patient suffers or has symptoms related with: liver diseases, alcoholism, brain diseases, limited salivation (in children), glaucoma, hyperactivity, renal or lung diseases, miastenia gravis, porphyrias, pregnancy or sleep apnea.

During treatment the following may be present: convulsions, fever, tremors, muscle weakness, reflex loss, intense asthenia, involuntary movements, unsteady breathing, mucose dryness (oral, conjunctive, nasal), eritematous skin, arterial hypotension, slow heart rate, mild mental alterations or even confusion and coma. Benzodiazepines cause dependency therefore they shall be used in short treatment periods. Upon treatment cancellation with benzodiazepines, it takes about three weeks for the body to get unaccustomed to it.

**Alprazolam** is a triazolobenzodiazepine which acts as anxiolytics and it is indicated for treatment and control of anxiety disorders, including panic with or without agoraphobia and generalized anxiety, as well as for short-term relief of characteristic symptoms of said disorders.

The exact action mechanism of Alprazolam has not been established up to now; however, it is known that 1,4-benzodiazepine group agents exert their action over CNS by attaching to stereospecific receptors found in several sites of said system. All benzodiazepines clinically cause a central nervous system depression related with dependent dose, in the range from minimum alterations and physical performance impairment up to hypnosis.

Alprazolam is rapidly absorbed after oral administration. Maximum plasma concentrations are obtained 1 to 2 hours after administering the corresponding dose. Plasma clearance half-life for Alprazolam is about 11.2 hours in healthy adults (with a range from 6.3 to 26.9 hours) and 16.2 hours in healthy elderly. Alprazolam is attached in 80% to human serum proteins (mainly to albumins. Alprazolam metabolism is performed by liver biotransformation through oxidative and glucuronization reactions, those which are carried out at liver level by microsomal oxidation, a process which is mediated by enzymatic system cytochrome P450 (CYP) through 3A4 isoenzyme. Its two main metabolites are: alpha-hydroxy-Alprazolam, having a biological activity which corresponds to about one half the therapeutic activity manifested by Alprazolam and one benzophenone, this being essentially inactive. Accumulated levels in plasma of these metabolites are extremely low. Alprazolam and its metabolites are mainly cleared through urine.

Alcoholism, liver failure and renal failure modify Alprazolam pharmacokinetics. Pharmacokinetic changes are also present in senile and obese patients, alcoholic patients with liver disease, healthy women taking oral contraceptives and patients with cimetidine treatment, those who show an extension in Alprazolam average half life. Alprazolam traverses the placental barrier and is excreted through breast milk. As other triazolobenzodiazepines, Alprazolam shows significant interaction with other drugs which are metabolized through P-450 hepatic cytochrome.

In any combination therapy, the potential for drug-drug interaction is present. Of specific significance is the potential interaction between Sertraline and Alprazolam since Alprazolam metabolism is catalyzed almost exclusively by P450 cytochrome, specifically by CYP3A4 isoenzyme.

The metabolism performed through CYP3A4 may be inhibited by a wide variety of components, *in vitro* evidence has shown that both Sertraline and its metabolite have the capability of inhibiting Alprazolam metabolism.

Clinically, Alprazolam metabolism inhibition may cause significant consequences since Alprazolam concentrations in stable state are known to be related with response and toxicity to concentrations higher than 60 ng/mL, then severe adverse events may be present such as memory failures, fatigue and sedation. In this way, the potential for drug interaction is clinically significant by coadministering Alprazolam and Sertraline.

The 5-HT reuptake selective inhibitor antidepressant agent used in the pharmaceutical composition subject of present invention is active principle: Sertraline, which is present in the formulation in a concentration range from 50.0 mg to 200.0 mg, being preferably used a concentration of about 50.0 mg. to 100.0 mg. per dose unit.

The anxiolytic agent belonging to the group of triazolobenzodiazepines used in the pharmaceutical composition subject of present invention is active principle: Alprazolam, which is present in the formulation in a concentration range from 0.25 mg. a 1.0 mg., being preferably used a concentration of about 0.25 mg. a 0.50 mg., per dose unit.

The pharmaceutical composition protected under present invention is formulated to be orally administered in a single dosage unit, in a capsule or tablet form, wherein the synergistic combination of active principles: Sertraline and Alprazolam is comprised, as well as pharmaceutically acceptable excipients.

Said pharmaceutical composition has been developed in order to provide a pharmaceutical alternative for control and treatment of diseases such as: depressive disorders and related pathologies, which provides significant advantages such as: lower concentrations of active principles comprised in formulation, lower administered doses, faster action, higher therapeutic effect efficacy, the satisfactory control of symptoms manifested by patients with depressive disorders, lower interaction risk among drugs (drug-drug) at liver stage and lower risks of adverse events to be present.

In order to assess the efficacy and tolerance of the pharmaceutical composition subject of the present invention, as well as the synergistic effect of the active principles Sertraline and Alprazolam combined in a single dosage unit, a comparative clinical study was performed wherein above mentioned active principles were separately administered, as well as a combination thereof.

### COMPARATIVE STUDY OF A SERTRALINE / ALPRAZOLAM COMBINATION

Some *in vitro* data show the inhibition of Alprazolam metabolism by Sertraline through CYP3A4, therefore this design was used to assess this *in vivo* potential in humans, especially to prove that by using lower dosages in combination better results are provided without inhibition alterations.

Inhibition of Alprazolam metabolism may clinically produce significant consequences since Alprazolam concentrations in steady state are known to be related with the response and toxicity to concentrations higher than 60 ng/mL, thus a frequency increase in adverse events such as failure in memory, fatigue and sedation may be present.

We performed a study on 30 healthy volunteer subjects using a double-blind design. Subjects with ages between 18 and 55 years were recruited. Exclusion criteria were: the use of substances with activity in CYP3A4 (quinidines), inhibitors (erythromycin), inducers (carbamazepines); consumption of illegal drugs (cocaine, heroin, tetrahydrocannabinoids); cigarettes, oral contraceptives, pregnancy, nursing, renal or liver disease history, obesity (>30% above ideal weight), abnormal biochemistry (electrolytes, liver or renal function) or hematologic (full count of blood cells).

Subjects were divided in 3 groups:
Group 1 received a combination of Alprazolam 1 mg. / Sertraline 50 mg.
Group 2 received a combination of Alprazolam 1 mg. / Sertraline 100 mg.
Group 3 received a combination of Alprazolam 0.25 mg. / Sertraline 50 mg.,
orally during 2 weeks of treatment.

After medicament delivery in each group, blood samples were taken at 0.5, 1, 1.5, 2, 2.5, 3, 4, 6, 8, 24 y 32 hours. In a term of 8 hours, researchers assessed the adverse effects of Alprazolam / Sertraline combinations in all dosages, such as: sedation, psychomotor failure and memory failure in each of the patients.

At the end of each study blood samples were taken for measuring the plasma concentrations in Alprazolam / Sertraline combinations.

### RESULTS.

All subjects completed the study (n=30), subject age was 30 year average with a range from 26 to 46 years. There were no reports of known drugs which interacted with CYP3A4 during the study term.

Alprazolam (Cmax, Tₘₐₓ, t_{1/2[β]}, AUC₀) kinetic parameters changed with combination. Cmax decreased compared to Alprazolam / Placebo administration (Table 1, 2 and 3).

**Table 1.**

| | Alprazolam 1 mg / Placebo (n=10) | Alprazolam 1 mg/. Sertraline 50 mg (n=10) |
|---|---|---|
| Cₘₐₓ (ng/mL)^{b} | 12.6 ± 7.5 | 8.4 ± 2.3 |
| Tₘₐₓ (hr)^{b} | 2.6 ± 1.4 | 2.5 ± 0.8 |
| t_{1/2} (hr)^{b} | 11.3 ± 2.2 | 12.3 ± 3.2 |
| AUC₀ (ng/mL-hr)^{b} | 166.8 ± 53.1 | 142.0 ± 27.0 |

| | | |
|---|---|---|
| C_{máx:} Alprazolam peak concentration; T_{máx:} Time for Alprazolam peak concentration; t_{1/2:} Clearance half-life; AUC_{0:} Time curve of baseline to infinite concentration. | | |

**Table 2.**

| | Alprazolam 1 mg/ Placebo (n=10) | Alprazolam 1 mg / Sertraline 100 mg (n=10) |
|---|---|---|
| Cₘₐₓ (ng/mL)^{b} | 12.6 ± 7.5 | 7.5 ± 1.3 |
| Tₘₐₓ (hr)^{b} | 2.6 ± 1.4 | 2.5 ± 1.2 |
| t_{1/2} (hr)^{b} | 11.3 ± 2.2 | 12.3 ± 5.4 |
| AUC₀ (ng/mL-hr)^{b} | 166.8 ± 53.1 | 130.3 ± 34.0 |

| | | |
|---|---|---|
| C_{máx:} Alprazolam peak concentration; T_{máx:} Time for Alprazolam peak concentration; t_{1/2}, Clearance half-life ; AUC_{0:} Time curve of baseline to infinite concentration. | | |

**Table 3.**

| | Alprazolam 1 mg/ Placebo | Alprazolam 0.25 mg/ Sertraline 50 mg. |
|---|---|---|
| | (n=10) | (n=10) |
| C_{máx} (ng/mL)^{b} | 12.6 ± 7.5 | 7.3 ± 1.1 |
| T_{máx} (hr)^{b} | 2.6 ± 1.4 | 2.3 ± 1.1 |
| t_{1/2} (hr)^{b} | 11.3 ± 2.2 | 12.1 ± 5.6 |
| AUC₀ (ng/mL-hr)^{b} | 166.8 ± 53.1 | 140.2 ± 25.4 |

| | | |
|---|---|---|
| C_{máx:} Alprazolam peak concentration; T_{máx:} Time for Alprazolam peak concentration; t_{1/2:} Clearance half-life; AUC_{0:} Time curve of baseline to infinite concentration. | | |

### Sertraline influence over Alprazolam adverse effects.

Interactions were not found between Sertraline and Alprazolam in DSST sedation assessments (Digit-Symbol Substitution Test) and in the scores for immediate and late memory in any of their parameters (peak effect, time of peak effect, and AUC) for any Sertraline dose.

Interaction effects were not observed either in Psychomotor Performance Manual parameters.

| | 1mg/50mg Alprazolam / Sertraline | 1mg/100mg Alprazolam /Sertraline | 0.25mg/50 mg Alprazolam / Sertraline |
|---|---|---|---|
| **Sedation** | | | |
| Peak effect | 60.6 | 76.4 | 59.3 |
| Peak effect time (hr.) | 2.7 | 2.2 | 2.0 |
| AUC (0-8 hr.) | 182.0 | 371.3 | 179.3 |
| **DSST** | | | |
| Peak effect | -19.0 | -41.4 | -18.7 |
| Peak effect time (hr.) | 1.2 | 2.0 | 1.2 |
| AUC (0-8 hr.) | 42.7 | 142.8 | 41.6 |
| **Performance Manual** | | | |
| Peak effect | -18.3 | -39.5 | 17.9 |
| Peak effect time (hr.) | 1.8 | 2.0 | 1.6 |
| AUC (0-8 hr.) | 53.2 | 117.1 | 53.0 |
| **Immediate Memory** | | | |
| Peak effect | -36.7 | -65.4 | -35.3 |
| Peak effect time (hr.) | 2.0 | 2.0 | 2.0 |
| AUC (0-8 hr.) | 134.2 | 298.3 | 133.3 |
| **Late Memory** | | | |
| **Peak effect** | -79.1 | -96.1 | -78.7 |
| Peak effect time (hr.) | 3.1 | 2.0 | 1.9 |
| AUC (0-8 hr.) | 398.3 | 514.5 | 397.3 |

| | | | |
|---|---|---|---|
| AUC: Area below the concentration-time curve; DSST: Digit-symbol substitution test; Peak effect is assessed through a change percentage from baseline to infinite. | | | |

Results demonstrate that Sertraline does not significantly alter Alprazolam kinetics, nor the adverse effects when these two drugs are administered in combination.

Although reasons for these reductions remain unexplainable, Cmax reduction and the absence of changes in other kinetic parameters may involve that Sertraline alters the absorption and/or distribution of Alprazolam without affecting its metabolism.

The conclusion is that by using active principles in combination with a single dose unit; with minimum doses of Alprazolam as 0.25 mg. and Sertraline 50 mg., the pharmaceutical composition is therapeutically effective, reducing even more the risk that adverse effects are manifested compared with the administration of said active principles in larger doses and separately.

### NOVELTY OF INVENTION

Having described the present invention, this is considered novel and therefore property is claimed under the contents of the following

## Claims

1. Pharmaceutical composition comprising a synergistic combination of a 5-HT reuptake selective inhibitor antidepressant agent and an anxiolytic agent belonging to the group of triazolobenzodiazepines, **characterized in that** the 5-HT reuptake selective inhibitor antidepressant agent is the active principle: Sertraline and the anxiolytic agent belonging to the group of triazolobenzodiazepines is active principle: Alprazolam, as well as pharmaceutically acceptable excipients, wherein said active principles are present in the formulation in a concentration range from 50.0 mg. to 200.0 mg. for Sertraline and from 0.25 mg. to 1.0 mg. for Alprazolam; which are formulated in a single dosage unit to be orally administered, the same which is indicated for control and treatment of depressive disorders and related diseases.

2. Pharmaceutical composition according to claim 1, **characterized in that** the 5-HT reuptake selective inhibitor antidepressant agent such as active principle: Sertraline, is present in the formulation in a concentration range from 50.0 mg. to 200.0 mg., being preferably used in formulation a concentration of 50.0 mg. to 100.0 mg., per dose unit.

3. Pharmaceutical composition according to claims 1 y 2, **characterized in that** the anxiolytic agent belonging to the group of triazolobenzodiazepines, such as the active principle: Alprazolam, is present in the formulation in a concentration range from 0.25 mg. to 1.0 mg., being preferably used in formulation a concentration of 0.25 mg. to 0.50 mg., per dose unit.

4. Pharmaceutical composition according to claims 1 to 3, **characterized in that** is formulated in a single dosage unit for oral administration in capsule or tablet form.

5. The use of the pharmaceutical composition according to claims 1 to 4, **characterized in that** is indicated for control and treatment of diseases such as: depressive disorders and related pathologies.
